**Europäisches Patentamt**

**European Patent Office**

**Office européen des brevets**

(11) Numéro de publication: **0 400 243**
**A1**

# (12) DEMANDE DE BREVET EUROPEEN

(21) Numéro de dépôt: **89401475.2**

(22) Date de dépôt: **30.05.89**

(51) Int. Cl.5: **A61M 31/00, A61L 31/00**

(43) Date de publication de la demande:
**05.12.90 Bulletin 90/49**

(84) Etats contractants désignés:
**AT BE CH DE ES FR GB GR IT LI LU NL SE**

(71) Demandeur: **Yakoun, Maurice Lucien**
**161, rue Pioch de Boutonnet**
**F-34000 Montpellier(FR)**

Demandeur: **Piedra, Christian**
**Camin de Las Argalas**
**F-34160 St Genies des Mourges(FR)**

(72) Inventeur: **Yakoun, Maurice Lucien**
**161, rue Pioch de Boutonnet**
**F-34000 Montpellier(FR)**
Inventeur: **Piedra, Christian**
**Camin de Las Argalas**
**F-34160 St Genies des Mourges(FR)**

(74) Mandataire: **Chauchard, Robert et al**
**c/o Cabinet Malémont 42, avenue du**
**Président Wilson**
**F-75116 Paris(FR)**

(54) **Elément prophylactique, tel que drain, doté d'une structure de diffusion in situ d'un produit thérapeutique, notamment d'un agent anti-microbien.**

(57) Elément prophylactique à usage thérapeutique, constitué d'un corps (1), tel qu'un drain, auquel est associée au moins une structure (3) renfermant ou susceptible de renfermer un produit thérapeutique, notamment un agent anti-microbien, cette structure étant en un matériau biologiquement compatible apte à laisser diffuser in situ ledit produit thérapeutique, caractérisé en ce que ladite structure (3) est une structure rapportée au corps (1) de l'élément prophylactique, qui est en outre subdivisée en sections individuelles (4) de rétention provisoire du produit thérapeutique à diffuser, ladite structure rapportée étant de préférence constituée par un chapelet (3) de petits éléments de diffusion (4) définissant lesdites sections individuelles et raccordés l'un à l'autre par des tubes de liaison (5).

FIG.1

## Elément prophylactique, tel que drain, doté d'une structure de diffusion in situ d'un produit thérapeutique, notamment d'un agent anti-microbien

La présente invention porte sur un élément prophylactique à usage thérapeutique constitué d'un corps, tel qu'un drain, auquel est associée au moins une structure renfermant ou susceptible de renfermer un produit thérapeutique, notamment un agent anti-microbien, cette structure étant en un matériau biologiquement compatible apte à laisser diffuser in situ ledit produit thérapeutique.

La pose de drains ou autres accessoires à usage médical du même type, s'implantant dans des cavités ou des conduits anatomiques du corps humain ou animal, se traduit dans un certain nombre de cas, par des infections locales, que l'on cherche à prévenir par des méthodes de prophylaxie sans toutefois obtenir des résultats très satisfaisants.

On connaît, par exemple, des éléments prophylactiques du type précité, en forme de cathéters dont la paroi est rendue micro-poreuse pour constituer à elle-seule la structure susceptible de renfermer et de laisser diffuser un agent anti-microbien, par contact avec un tissu ou un liquide biologique de l'organisme.

Il s'avère toutefois qu'en raison de sa répartition très aléatoire à l'intérieur de la paroi du cathéter, l'agent anti-microbien est libéré souvent de façon trop imprécise ou insuffisamment uniforme pour être en mesure de prévenir ou de combattre efficacement et en toutes circonstances des infections locales. De surcroît, l'introduction de l'agent anti-microbien dans la paroi des cathéters prophylactiques connus n'est pas réalisable de façon simple et se fait le plus souvent dans le cours de la fabrication par extrusion du cathéter ce qui rend impossible tout rechargement et donc toute réutilisation à but prophylactique du cathéter. Enfin, les cathéters connus ne peuvent recevoir dans leur paroi qu'un seul type d'agent anti-microbien alors que, dans certains cas, il peut être souhaitable de pouvoir diffuser parallèlement deux ou trois agents ayant des effets prophylactiques différents.

La présente invention se propose de remédier à ces inconvénients et, pour ce faire, elle a pour objet un élément prophylactique, en particulier un drain, du type spécifié en introduction, qui se caractérise en ce que ladite structure est une structure rapportée au corps de l'élément prophylactique, qui est en outre subdivisée en sections individuelles de rétention provisoire du produit thérapeutique à diffuser, notamment de l'agent anti-microbien.

Lorsque l'élément prophylactique selon l'invention est utilisé pour une asepsie, la diffusion de l'agent anti-microbien se fera ainsi à hauteur de chacune des sections individuelles de la structure rapportée, c'est-à-dire de façon pratiquement ponctuelle et donc précise, dans le cas de risques d'infections très localisées, ou uniforme, dans le cas de risques d'infections plus étendues.

En outre, en réalisant lesdites sections individuelles sous la forme d'éléments creux à paroi diffusante, constituant de petits réservoirs, on disposera d'une structure rechargeable à volonté.

Selon un mode de réalisation préféré, ladite structure rapportée est constituée par un chapelet de petits éléments de diffusion qui peuvent être avantageusement réalisés sous la forme de billes offrant la plus grande surface de diffusion possible pour l'agent anti-microbien.

Selon une autre caractéristique de l'invention, les éléments de diffusion sont raccordés l'un à l'autre par des tubes de liaison, qui permettent de charger facilement les éléments de diffusion en agent anti-microbien, à l'aide par exemple d'une seringue transperçant la paroi de ces tubes.

Par ailleurs, les tubes de liaison des éléments de diffusion sont, de préférence, excentrés par rapport à ces derniers pour pouvoir être directement solidarisés au corps et permettre ainsi une fixation simple du chapelet de billes sur ce dernier, ne nécessitant pas l'utilisation de moyens de support intermédiaires.

Selon un second mode de réalisation de l'invention, la structure rapportée peut en variante présenter la forme d'un cylindre compartimenté dans le sens de sa longueur.

Par ailleurs, les sections individuelles de la structure rapportée, notamment les billes du premier mode de réalisation ou les compartiments du cylindre constituant le second, peuvent être réalisées, soit en un matériau diffusant stable, par exemple un silicone, soit en un matériau résorbable, par exemple un plâtre biologiquement compatible. Dans le premier cas, en réalisant de préférence lesdites sections individuelles sous une forme creuse, on disposera d'une structure rechargeable à volonté et donc avantageusement réutilisable. Dans le second cas, la diffusion de l'agent anti-microbien sera grandement favorisée quand elle se fait par élution, comme cela est souvent le cas.

Quand le corps de l'élément prophylactique selon l'invention est un drain, la structure rapportée est de préférence fixée sur sa face intérieure et la paroi du drain est par ailleurs percée de micro-perforations assurant la diffusion de l'agent anti-microbien de l'autre côté de cette paroi.

Enfin, l'invention permet avantageusement la mise en place de plusieurs structures rapportées sur le corps de l'élément prophylactique, pour permettre la diffusion en parallèle de plusieurs agents anti-microbiens ayant des propriétés différentes.

Deux modes de réalisation de l'élément prophylactique selon l'invention vont maintenant être décrits plus en détail, uniquement à titre d'exemples non-limitatifs, en référence aux dessins annexés dans lesquels :

- la figure 1 est une vue en perspective, avec arrachement partiel, d'un élément prophylactique conforme au premier mode de réalisation,

- la figure 2 est une vue en coupe longitudinale verticale de cet élément prophylactique,

- la figure 3 est un agrandissement d'une partie de la figure 2,

- la figure 4 représente schématiquement, selon une vue en bout, un développement de l'élément prophylactique des figures 1 à 3, et

- la figure 5 est une vue analogue à la figure 3, illustrant le deuxième mode de réalisation de l'invention.

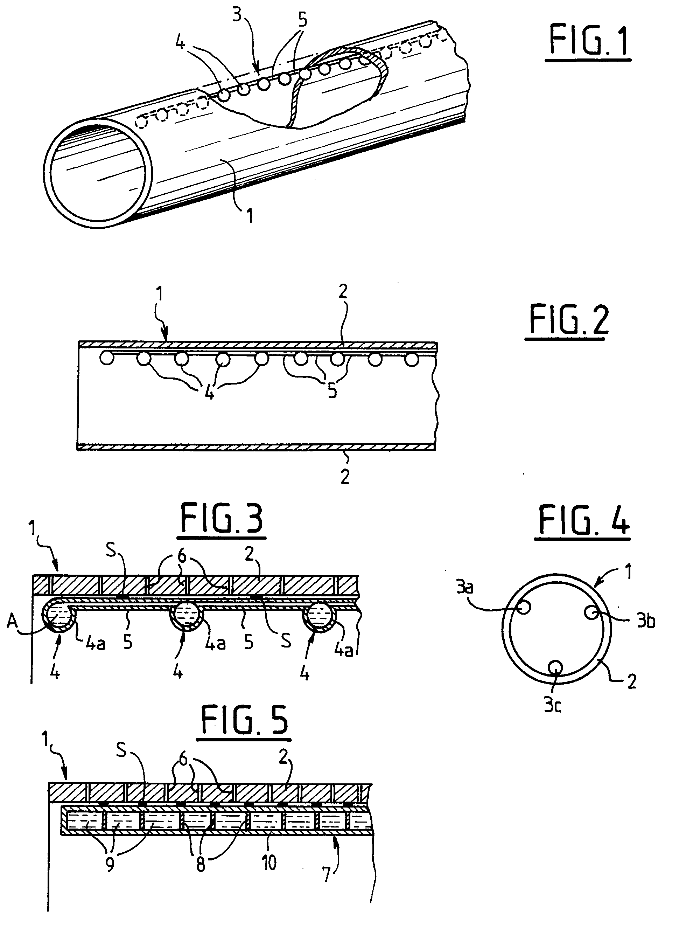

L'élément prophylactique visible sur la figure 1, se compose d'un long corps flexible 1, qui est ici un drain tubulaire dont la paroi 2 porte sur sa face intérieure, un chapelet 3 de petites billes 4 s'étendant le long d'une génératrice du drain 1, c'est-à-dire dans sa direction longitudinale.

Comme le font mieux ressortir les figures 2 et 3, les billes 4 sont creuses et communiquent successivement entre elles par de courts tubes de liaison 5, lesquels sont alignés sur un même axe sensiblement tangentiel à toutes les billes pour pouvoir constituer en même temps, pour le chapelet 3, des points de fixation directe au drain 1. Le chapelet 3 de billes, ici réalisé en un polymère stable et bio-compatible, tel qu'un silicone, un polytétrafluoroéthylène, un polyuréthane, etc... est plus précisément solidarisé, à la paroi 2 du drain elle-même constituée d'un élastomère bio-compatible, par un soudage ultrasonique par points S pratiqué au niveau des tubes 5. Toute autre technique de fixation, comme un collage, est cependant envisageable à cette fin.

Dans l'élément prophylactique représenté, les billes 4 sont remplies d'un agent anti-microbien A, et à cette fin, on peut avantageusement se servir des tubes 5 dans lesquels ce dernier sera alors injecté, par exemple, à l'aide d'une seringue.

Dès lors, une fois que le drain 1 est implanté par exemple dans une cavité du corps humain qu'il faut ponctionner, chaque bille, dont la paroi 4a est micro-poreuse, libérera progressivement la quantité d'agent anti-microbien qu'elle contient et celui-ci, en passant par des micro-perforations 6 dont est dotée la paroi 2 du drain 1, viendra de l'autre côté se déposer sur la zone adjacente de la paroi de la cavité pour y exercer son action prophylactique. L'utilisation des billes 4 réparties à intervalles égaux sur un tronçon au moins du drain 1, permet une diffusion contrôlée uniforme ou ponctuelle de l'agent anti-microbien. Par ailleurs, une fois que les billes se sont complètement vidées, elles peuvent facilement être rechargées en agent anti-microbien, de la manière indiquée ci-dessus, au travers des tubes 5.

La figure 4 montre également que plusieurs chapelets de billes, tels que 3a,3b,3c identiques au chapelet 3 de la figure 1, peuvent être fixés parallèlement entre eux et de la même façon que ce dernier, à l'intérieur du drain 1. Ces chapelets de billes 3a,3b,3c, peuvent être remplis d'agents anti-microbiens de natures différentes pour prévenir en des zones distinctes de la cavité dans laquelle le drain est introduit, des infections de types différents.

La figure 5 illustre un deuxième mode de réalisation de l'élément prophylactique selon l'invention, dans lequel le chapelet 3 ou chaque chapelet 3a,3b,3c du premier est remplacé par un long cylindre creux 7 en silicone micro-poreux, subdivisé, dans le sens de sa longueur, par des cloisons étanches 8 délimitant ainsi des compartiments individuels 9 qui jouent le rôle des billes 4, et, tout comme ces dernières, peuvent être remplis, au travers de la paroi extérieure perforable 10 du cylindre 7, d'un agent anti-microbien. Pour le reste, on retrouve dans ce deuxième mode de réalisation de l'invention, l'ensemble des caractéristiques décrites ci-dessus en liaison avec le chapelet 3 du premier, notamment quant au positionnement du cylindre 7, dans le sens longitudinal du drain 1, et à son mode de fixation à l'intérieur de ce dernier.

On peut bien entendu utiliser dans l'élément prophylactique selon l'invention tous les agents anti-microbiens actuellement connus, et notamment les pénicillines, les céphalosporines, les aminosides, les tétracyclines, le chloramphénicol et ses dérivés, les macrolides, les lincomycines, les rifamycines, les polyémyxidines, les sulfamides, les quinolones, les nitrofuranes, les antifongiques, la flucytosine, les imipénèmes, etc...

Il va de soi également que l'invention peut être appliquée à des drains présentant une autre forme que celle décrite, et en particulier à des drains en forme de lame, ainsi qu'à d'autres éléments à usage médical du même type, comme des cathéters.

Par ailleurs, l'élément prophylactique selon l'invention peut être utilisé pour mettre en oeuvre dans le corps humain des actions thérapeutiques autres qu'une prophylaxie anti-microbienne et dans ce cas bien entendu les billes 4 ou les compartiments 9 seront chargés du produit thérapeutique approprié.

Dans l'élément prophylactique selon l'invention, on peut en variante réaliser les billes 4 du premier mode de réalisation et les compartiments 9 du deuxième sous la forme de petits éléments pleins, dans lesquels l'agent anti-microbien ou autre produit thérapeutique sera introduit lors de la polymérisation de leur matière constitutive et se diffusera in situ, par élution dans le liquide biologique circulant dans l'élément prophylactique. Il est même possible de les réaliser, sous une forme creuse ou pleine, en un matériau biologiquement résorbable comme le plâtre ou un ciment acrylique, qui présentent la propriété d'être très bien tolérés par l'organisme, et dans ce cas, l'agent anti-microbien ou autre produit thérapeutique sera introduit dans les billes lors de la fabrication, par exemple par moulage, du chapelet 3 ou du cylindre 7. La possibilité de résorption du matériau constitutif des billes 4 ou des compartiments 9 favorise la diffusion par élution du produit thérapeutique.

Pour fabriquer les chapelets de billes 3 ou les cylindres compartimentés 7 de l'élément prophylactique selon l'invention, lorsque ceux-ci sont en une matière synthétique, on peut procéder à l'aide de techniques connues de moulage ou d'extrusion-soufflage, éventuellement en réalisant plusieurs éléments assemblés ensuite par soudage ou collage.

En conclusion, l'utilisation des éléments prophylactiques selon l'invention dans le cas notamment où il s'agit de drains, permet d'empêcher toute prolifération de micro-organismes dans le périmètre de la zone où ils sont utilisés, et ce pendant une période de 8 à 10 jours. En fait, quand il est employé comme drain, l'élément prophylactique de la présente invention permet de supprimer toutes les complications post-opératoires, notamment en chirurgie osseuse ou viscérale.

Bien entendu, les éléments prophylactiques selon l'invention sont utilisables indifféremment en médecine humaine ou vétérinaire.

## Revendications

1. Elément prophylactique à usage thérapeutique, constitué d'un corps (1), tel qu'un drain, auquel est associée au moins une structure (3,7) renfermant ou susceptible de renfermer un produit thérapeutique, notamment un agent anti-microbien, cette structure étant en un matériau biologiquement compatible apte à laisser diffuser in situ ledit produit thérapeutique, caractérisé en ce que ladite structure (3,7) est une structure rapportée au corps (1) de l'élément prophylactique, qui est en outre subdivisée en sections individuelles (4,9) de rétention provisoire du produit thérapeutique à diffuser.

2. Elément prophylactique selon la revendication 1, caractérisé en ce que ladite structure rapportée est constituée par un chapelet (3) de petits éléments de diffusion (4) définissant lesdites sections individuelles.

3. Elément prophylactique selon la revendication 2, caractérisée en ce que les éléments de diffusion (4) sont raccordés l'un à l'autre par des tubes de liaison (5).

4. Elément prophylactique selon la revendication 3, caractérisée en ce que les tubes de liaison (5) des éléments de diffusion (4) sont excentrés par rapport à ces derniers et sont directement solidarisés au corps (1).

5. Elément prophylactique selon l'une quelconque des revendications 2 à 4, caractérisé en ce que les éléments de diffusion sont en forme de petites billes (3).

6. Elément prophylactique selon la revendication 1, caractérisé en ce que ladite structure rapportée présente la forme d'un cylindre (7) compartimenté dans le sens de sa longueur, les compartiments (9) de ce cylindre constituant lesdites sections individuelles.

7. Elément prophylactique selon l'une quelconque des revendications 1 à 6, caractérisé en ce que lesdites sections individuelles (4,9) de la structure rapportée (3,7) sont réalisées en un matériau diffusant stable.

8. Elément prophylactique selon l'une quelconque des revendications 1 à 6, caractérisé en ce que lesdites sections individuelles (4,9) de la structure rapportée (3,7) sont réalisées en un matériau résorbable.

9. Elément prophylactique selon l'une quelconque des revendications 1 à 8, caractérisé en ce que lesdites sections individuelles (4,9) de la structure rapportée (3,7) sont réalisées sous la forme d'éléments creux à paroi diffusante.

10. Elément prophylactique selon l'une quelconque des revendications 1 à 9, dont le corps (1) est un drain, caractérisé en ce que ladite structure rapportée (3,7) est fixée sur la face intérieure du drain dont la paroi (2) est dotée de micro-perforations (6).

11. Elément prophylactique selon l'une des revendications 1 à 10, caractérisé en ce que son corps (1) porte plusieurs structures rapportées (3a,3b,3c).

FIG.1

FIG.2

FIG.3

FIG. 4

FIG. 5

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | FR-A-2 608 931 (KLOTZ)<br>* abrégé; figure 1 *<br>--- | 1-11 | A 61 M 31/00<br>A 61 L 31/00 |
| X | EP-A-0 207 250 (GEBRÜDER SULZER AG)<br>* abrégé; figure 1; colonne 4, lignes 28-45 *<br>--- | 1-2,6-7,9,11 | |
| X | DE-A-3 115 763 (LENHARD)<br>* abrégé; figure 1; page 11, lignes 8-11; page 12, lignes 9-32 *<br>--- | 1-2,6-9,11 | |
| A | EP-A-0 306 389 (HUTCHINSON)<br>* abrégé; figure 1 *<br>--- | 3-5 | |
| A | WO-A-8 203 174 (MERCK)<br>* page 1, lignes 3-7; figures 1,10 *<br>----- | 3-5 | |

DOMAINES TECHNIQUES RECHERCHES (Int. Cl.5)

A 61 M
A 61 L
A 61 F

Le présent rapport a été établi pour toutes les revendications

| Lieu de la recherche | Date d'achèvement de la recherche | Examinateur |
|---|---|---|
| LA HAYE | 30-01-1990 | MIR Y GUILLEN V. |